# FASCICULE DE BREVET EUROPEEN

(11) **EP 0 644 211 B1**
(45) Date de publication et mention de la délivrance du brevet: **24.09.1997**
(21) Numéro de dépôt: 94401653.4
(22) Date de dépôt: 19.07.1994
(51) Int. Cl.: C08F 220/06, C08F 2/32, A61L 15/00

(54) **Polymères acryliques superabsorbants à propriétés améliorées et leur procédé d'obtention**
Superabsorbierende Acryl-Polymere mit verbesserten Eigenschaften und Verfahren zu deren Herstellung
Superabsorbant acrylic polymers with better properties and process for their preparation

(30) Priorité: 21.09.1993 FR 9311231
(43) Date de publication de la demande: 22.03.1995
(73) Titulaire: ELF ATOCHEM S.A., 92800 Puteaux, Hauts de Seine (FR)
(72) Inventeur: Rebre, Shu Rong, F-94300 Vincennes (FR); Collette, Christian, F-75005 Paris (FR); Guerin, Thierry, F-93100 Montreuil (FR)
(74) Mandataire: Haicour, Philippe

(56) Documents cités:
- EP-A- 0 325 065
- EP-A- 0 441 507

## Description

La présente invention a trait aux polymères susceptibles d'absorber d'importantes quantités d'eau ou de fluides aqueux, et plus précisément aux polymères acryliques que l'on obtient par les procédés de polymérisation en suspension inverse.

Ces matériaux ont de nos jours reçu de larges applications industrielles, en particulier dans la fabrication d'articles sanitaires pour l'absorption et la rétention de liquides corporels.

La production de poudres de polyacrylates superabsorbants en suspension inverse a été décrite dans le brevet français FR 2.367.083 (K_{AO} S_{OAP}). Elle consiste à provoquer la polymérisation d'un acrylate en solution aqueuse préalablement mis en suspension dans un solvant aliphatique grâce à un agent émulsifiant d'HLB compris entre 3 et 6 et en l'absence d'agent de réticulation. On a ensuite amélioré les performances des poudres polymères grâce à l'utilisation d'un agent émulsifiant d'HLB compris entre 8 et 12 (EP 36463, Seitsu Kagaku Co. Ltd). La différence de résultat la plus apparente entre ces deux méthodes est qu'elles conduisent à des poudres polymères, pour la première sous forme de sphères, pour la seconde sous forme de particules à forme plus tourmentée.

Un perfectionnement important a consisté à procéder en deux temps, comme décrit dans le brevet européen EP 0441507, à savoir dans une première étape, à former un gel par polymérisation d'une solution acrylique en suspension inverse, puis à lui faire absorber une seconde charge de monomère, dont on provoque la polymérisation au sein même du gel précédemment formé.

Ces méthodes ne permettent pas d'atteindre des poudres polymères qui répondent correctement aux besoins de certains secteurs importants de l'industrie, parce que les poudres absorbantes que l'on obtient ainsi ont un temps de prise en gel (T.P.G.), trop long, ou qu'alors si elles présentent un T.P.G. acceptable, c'est au détriment de leurs autres propriétés physiques ou de leur teneur en extractibles.

La présente invention remédie à ces inconvénients, avec un procédé de préparation des poudres polymères absorbantes présentant à la fois un temps de prise en gel faible, une succion capillaire élevée, une forte force de gel et une faible teneur en extractibles. Le procédé selon l'invention est un procédé par double polymérisation (on entend par procédé par double polymérisation, un procédé comprenant une première étape de polymérisation d'une première charge (charge I) de monomère en solution aqueuse mise en suspension inverse dans un solvant organique grâce à un tensioactif, dont HLB est élevé (HLB de 8 à 12), et une deuxième étape au cours de laquelle on fait absorber par le gel polymère obtenu dans la première étape une deuxième charge (charge II) de monomère que l'on fait ensuite polymériser au sein de ce gel.

L'originalité du procédé selon la présente invention consiste dans la combinaison des éléments suivants :
- présence d'un réticulant dans la première charge de polymérisation,
- augmentation importante de la quantité de charge de monomère acrylique que l'on fait absorber au gel au cours de la seconde étape du procédé, et
- introduction d'un tensioactif d'HLB bas dans le milieu de réaction, très précisément après l'absorption de la deuxième charge de monomère par le gel et avant sa polymérisation.

Le tensioactif à utiliser ici après l'absorption de la charge II et avant sa polymérisation doit avoir un HLB approximativement de 2 à 5. Des esters d'acides gras et de glycérol, de sorbitol ou de saccharose répondent à cette caractéristique. On préfère le di/tristéarate de saccharose.

Dans les procédés par double polymérisation de l'art antérieur, les quantités respectives de monomère, et plus précisément de monomère acrylique, mises en oeuvre dans la première et la seconde étape, que l'on peut représenter par le rapport "charge II"/"charge I" étaient limitées à une valeur pondérale approximative de 1:2. Il était impossible d'aller au-delà, parce qu'on assistait alors à une prise en masse du contenu du réacteur. Selon l'invention, ce rapport de charge est de 1 à 1,2 et sa mise en oeuvre est possible à condition d'introduire un réticulant dans la charge I. Il est possible d'utiliser à cette fin tout composé susceptible de réticuler les polymères d'acides carboxyliques α,β-insaturés ou de leurs dérivés. De tels produits sont bien connus de l'homme du métier, qui en trouvera facilement des listes bien fournies, par exemple dans la demande de brevet européen EP 0176664. Des agents préférés de réticulation sont les dérivés bis acryliques, en particulier le N,N-méthylènebisacrylamide et les composés du type diglycidyléther, en particulier l'éthylène glycol diglycidyléther. L'introduction d'un tel agent de réticulation dans la charge II est également bénéfique.

Dans les procédés de polymérisation en suspension inverse en deux étapes selon un art antérieur classique, il était nécessaire d'effectuer l'absorption de la charge II à basse température. Sinon, l'agent tensioactif utilisé au cours de la première étape pour mettre en suspension inverse la solution aqueuse acrylique restait actif, et favorisait la mise en suspension inverse de la charge II au détriment de son absorption par le gel préalablement formé. C'était une contrainte d'importance dans la conduite du procédé industriel. Le procédé selon l'invention est en ce point beaucoup moins sensible ; on peut travailler à des températures proches de l'ambiante, voire des températures aussi élevées que 40°C pour autant que l'on choisisse judicieusement le tensioactif de la première étape. C'est un gros avantage au plan industriel, car après la première polymérisation le contenu du réacteur est habituellement à environ 80°C et son refroidissement est une opération longue: on raccourcit considérablement le cycle de production, et par voie de conséquence on améliore significativement la productivité, en n'ayant à le ramener qu'à 40°C plutôt qu'à 20°C comme dans l'art antérieur.

On améliore encore le procédé en augmentant la teneur du gel en matière acrylique, c'est-à-dire en augmentant le rapport de phase aqueuse sur phase organique au cours de la première étape. Une telle action n'est pas réalisable selon l'art antérieur parce la suspension inverse réalisée au cours de la première étape présente des signes d'instabilité dès que le rapport phase aqueuse/phase organique atteint 0,4-0,6. Il se trouve que selon le procédé de l'invention, on contrôle mieux la polymérisation du fait de l'utilisation d'un réticulant : on peut dès lors opérer avec des rapports phase aqueuse/phase organique élevés allant de 0,7 à 1,1.

Grâce au nouveau procédé décrit, on peut obtenir des polymères superabsorbants présentant une combinaison de propriétés uniques, à savoir :
- un temps de prise en gel compris entre 15 et 40 secondes,
- une force de gel supérieure à 2.800 Pascals,
- une succion capillaire sous 2 kPa supérieure à 30 ml/g,
- une succion capillaire sous 5 kPa supérieure à 20 ml/g,
- une capacité d'absorption supérieure à 50 g/g,
- un taux d'extractibles inférieur à 10 %,
- une teneur en fines inférieure à 10 %,
- une rétention supérieure à 28 g/g.

Ce sont des produits nouveaux, qui constituent également un objet de la présente invention. Ils trouvent une application immédiate dans les articles, notamment pour l'hygiène féminine et l'incontinence d'adultes.

### Exemples

Dans les exemples qui suivent, on distingue les séquences :
a) de préparation de la phase solvant,
b) de préparation de la phase aqueuse de monomère (charge I)
c) de mise en suspension du monomère et polymérisation I,
d) de préparation de la phase aqueuse de monomère (charge II),
e) d'absorption de la charge II et de polymérisation II,
f) de séparation du polymère.

On apprécie les propriétés des poudres superabsorbantes ainsi obtenues selon des méthodes connues de l'homme de l'art, que l'on rappelle brièvement ci-après.

*Capacité d'absorption de solution saline* : on disperse 1 gramme de poudre superabsorbante dans 200 grammes de solution à 0,9 % de chlorure de sodium et on maintient sous agitation pendant deux heures. On filtre sur un tamis à maille de 75µm légèrement incliné (15°), et on pèse après 30 minutes. Si p est le poids de poudre gonflée retenue sur le filtre, la capacité d'absorption se mesure par p-1.

*Temps de prise en gel (T.P.G.)* : dans un bécher de 200 ml (φ intérieur = 55 mm), muni d'un agitateur magnétique tournant à 600 t/min, on introduit 100 ml d'une solution à 0,9 % de chlorure de sodium à la température de 20°C, puis rapidement 3 grammes de poudre superabsorbante. On chronomètre le temps nécessaire à la disparition du vortex qui s'était formé dans le liquide sous l'effet de l'agitation. Ce temps exprimé en secondes est le T.P.G.

*Force de gel* : la méthode suivie consiste à placer 58 g d'une solution aqueuse de chlorure de sodium à 0,9 % dans un bécher muni d'une agitation magnétique, d'y ajouter 2 grammes de poudre superabsorbante et de poursuivre l'agitation jusqu'à ce par effet de l'évolution rhéologique de son contenu, la surface du bécher devienne horizontale par disparition du vortex initialement formé sous l'effet de l'agitation. On mesure alors le module élastique du gel, exprimé ici en N/m², au moyen du "neo-crud-meter" modèle M-302, commercialisé par Electric Corporation of Japan.

*Succion capillaire sous pression* : le principe est celui de l'absorption par capillarité d'une solution saline par une poudre superabsorbante placée entre deux feuilles de non-tissé et soumise au travers du non-tissé à une pression soit de 2kPa, soit de 5 kPa. Le dispositif expérimental et du mode opératoire suivis sont décrits, par exemple, dans le brevet français FR 2602775.

*Taux d'extractibles* : on effectue cette mesure par dosage potentiométrique à point final absolu de la teneur en acide polyacrylique, polyacrylate de sodium et divers monomères extractibles par l'urine synthétique représentée par une solution aqueuse comprenant 15 g d'une solution à 1% de Triton® X 100 (Rohm & Haas), 60 g de chlorure de sodium, 1,8 g de chlorure de calcium dihydraté, 3,6 g de chlorure de magnésium hexahydraté et 6.000 ml d'eau distillée. On procède par dosage en retour sur la solution obtenue par macération de 1,2 g de poudre superabsorbante dans 225 ml d'urine synthétique pendant 16 heures à température ambiante, et sous agitation mécanique de 500 tours/minute, puis filtration sur tamis à la maille de 50 µm.

### Exemple 1 (conforme à l'art antérieur selon EP 0441507)

*Séquence a)*
   Dans un réacteur d'un litre muni d'un dispositif d'introduction de réactifs solides ou liquides, d'un agitateur, d'un système de balayage par gaz neutre, d'une sonde de température et d'un dispositif de chauffage/réfrigération, on dissout à 80°C et sous agitation de 800 tours/minute et sous courant d'azote de 0,2 litre/minute, 0,97 g de monolaurate de sorbitan (HLB = 8,6), un produit commercialisé par Nippon Oil and Fats Co. sous le nom de LP20 R dans 385 g d'heptane. Le mélange est ramené à 30°C
*Séquence b)*
   A part, on neutralise 92 g d'une solution aqueuse à 80 % en poids d'acide acrylique par 152,6 g de lessive de soude à 20,1 %. On ajoute 2,2 g d'une solution aqueuse à 5 % de persulfate de potassium. Cette opération est conduite à la température d'environ 15°C.
*Séquence c)*
   Le réacteur étant maintenu sous agitation de 800 tours/minute et sous balayage d'azote à raison de 0,2 litre/minute, on y introduit peu à peu la phase aqueuse précédemment préparée qui passe en suspension inverse dans l'heptane. La température est portée à 70°C pour provoquer la polymérisation ; elle est maintenue à ce niveau pendant 30 minutes. La température est alors ramenée à 10°C.
*Séquence d)*
   Pendant le déroulement de l'opération précédente, on procède à part à la neutralisation de 46 g de solution aqueuse à 80 % en poids d'acide acrylique par 76,3 g de lessive de soude à 20,1 %, puis on ajoute 1,1 g d'une solution aqueuse à 5 % de persulfate de potassium. On a procédé à la préparation de cette phase aqueuse qui constitue la charge II de monomère à une température d'environ 10°C.
*Séquence e)*
   L'agitation dans le réacteur étant maintenue à 800 tours/minute et le balayage d'azote à 0,2 litre/minute, on y introduit en environ 15 minutes la charge II, on maintient encore l'agitation pendant 5 minutes, après quoi la température est portée à 70°C pour engager la seconde phase de polymérisation. On laisse la polymérisation se poursuivre pendant une demi-heure.
*Séquence finale f)*
   On élimine l'heptane et la majeure partie de l'eau par distillation. On ajoute alors au contenu du réacteur 8,3 g d'une solution aqueuse à 2 % d'éthylène glycol diglycidyléther (un composé commercialisé sous le nom de E100 par Nippon Oil and Fats Co.) et on poursuit séchage.

On obtient ainsi 97,2 grammes de poudre superabsorbante dont la taille des particules est inférieure à 800µm. Le rendement exprimé par rapport à l'acide acrylique est 72 %.

### Exemple 2 (comparatif)

*Séquence a)*
   Identique à exemple 1.
*Séquence b)*
   Identique à exemple 1.
*Séquence c)*
   Identique à exemple 1.
*Séquence d)*
   Pendant le déroulement de l'opération précédente, on procède à part à la neutralisation de 92 g de solution aqueuse à 80 % en poids d'acide acrylique par 139,4 g de lessive de soude à 20,1 %, puis on ajoute 5,5 g d'une solution aqueuse à 1 % de persulfate de potassium. On a, comme dans l'exemple précédent, procédé à la préparation de cette phase aqueuse qui constitue la charge II de monomère à une température d'environ 10°C.
*Séquence e)*
   L'agitation dans le réacteur étant maintenue à 800 tours/minute et le balayage d'azote à 0,2 litre/minute, on y introduit en environ 15 minutes la charge II, on maintient encore l'agitation pendant 5 minutes. On introduit alors 7,36 g d'une solution à 10 % de di/tristéarate de saccharose, un produit commercialisé par Mitsubishi Chemical Food Ind. Co. sous le nom de S370, après quoi, comme dans l'exemple précédent, la température est portée à 70°C pour engager la seconde phase de polymérisation. On laisse la polymérisation se poursuivre pendant une demi-heure.
*Séquence finale f)*
   Identique à exemple 1.

On obtient ainsi 129 grammes de poudre superabsorbante. Le rendement exprimé par rapport à l'acide acrylique est 71,3 %.

Le tableau ci-dessous permet de comparer les résultats du présent essai à ceux de l'essai précédent.

| **ESSAI** | **Exemple 1** | **Exemple 2** |
|---|---|---|
| **Capacité d'absorption (g/g)** | 57 | 56 |
| **Rétention 30 (g/g)** | 27 | 26 |
| **T.P.G (s)** | 5 | 6 |
| **Force de gel (Pa)** | 2700 | 2500 |
| **Extractibles (%)** | 15 | 17 |
| **Succion sous 2kPa (ml/g)** | 22 | 21 |
| **Succion sous 5kPa (ml/g)** | 17 | 17 |
| **Diamètre médian (um)** | 237 | 240 |
| **< 100 um (%)** | 18,7 | 16 |

### Exemples 3 et 4

On améliore les résultats obtenus dans l'exemple 2 en diminuant considérablement la quantité de solvant organique dans laquelle la charge I est mise en suspension inverse. II est possible de réaliser ces conditions en introduisant un réticulant dans la charge I. Les séquences réalisées sont les suivantes :
*Séquence a)*
   Comme dans l'exemple 2, mais la vitesse de rotation de l'agitateur est ramenée à 500 tours/minute, la quantité de n-heptane n'est que de 264 g, et le dosage en monolaurate de sorbitan est de 0,5 g.
*Séquence b)*
   Comme dans l'exemple 2, mais on ajoute en plus 0,92 g d'une solution à 2 % d'éthylène glycol diglycidyléther (E100).
*Séquence c)*
   Comme dans l'exemple 2. Toutefois, en fin d'opération, la température du réacteur est de 10°C pour l'exemple 3, et de 40°C pour l'exemple 4.
*Séquence d)*
   Comme dans l'exemple 2, à la différence que la charge II contient en outre 0,92 g d'une solution à 2 % d'éthylène glycol diglycidyléther (E100). En fin de neutralisation, les températures sont ramenées à 10°C pour l'exemple 3 et à 40°C pour l'exemple 4.
*Séquence e)*
   Cette séquence se déroule comme dans l'exemple 2, y compris l'introduction de di/tristéarate de saccharose.
*Séquence finale f)*
   Identique à exemple 2.

On obtient ainsi respectivement 155 grammes de poudre superabsorbante dans l'exemple 3 et 164,4 grammes dans l'exemple 4, ce qui correspond à des rendements respectifs en acide acrylique de 87 % et 90,1 %.

Les résultats obtenus sont rassemblés dans le tableau ci-dessous et comparés aux résultats de l'exemple 1.

| **EXEMPLES** | **1** | **3** | **4** |
|---|---|---|---|
| **Température II (°C)** | 10 | 10 | 40 |
| **SAP (g)** | | | |
| - *Quantité obtenue* | 97,2 | 155 | 164,4 |
| - *Quantité théorique* | 135,7 | 180,9 | 180,9 |
| **Capacité d'absorption (g/g)** | 57 | 56 | 57 |
| **Rétention 30 (g/g)** | 27 | 30 | 30 |
| **T.P.G (s)** | 5 | 25 | 21 |
| **Force de gel (Pa)** | 2700 | 2850 | 3000 |
| **Extractibles (%)** | 15 | 8 | 6,4 |
| **Succion sous 2kPa (ml/g)** | 22 | 32 | 37 |
| **Succion sous 5kPa (ml/g)** | 17 | 21 | 23 |
| **Diamètre médian** | 237 | 255 | 220 |
| **Passant à 100µm (%)** | 18,7 | 7,5 | 8,4 |

### Exemple 5 (contre-exemple)

On reproduit les conditions de l'exemple 4, à la différence essentielle qu'au cours de la séquence e, on omet l'introduction de di/tristéarate de saccharose. Dès que l'on déclenche la polymérisation en montant la température, le réacteur se prend en masse et l'essai doit être interrompu.

### Exemple 6

Variante de l'exemple 4 à la différence duquel dans la séquence a, la vitesse de rotation est ramenée à 300 tours/minute et que le dosage en monolaurate de sorbitan est maintenant de 0,67 g. Le déroulement de l'essai est très comparable à celui de l'exemple 4, tant en qualité de poudre absorbante qu'en rendement, ainsi qu'en témoignent les chiffres rapportés ci-dessous.

| **EXEMPLE** | **N°4** | **N°6** |
|---|---|---|
| **Quantité obtenue** | 164,4 | 155,7 |
| **Rendement (%)** | 91 | 86 |
| **Capacité d'absorption (g/g)** | 57 | 55 |
| **Rétention 30 (g/g)** | 30 | 28 |
| **T.P.G. (s)** | 21 | 28 |
| **Force de gel (Pa)** | 3000 | 3000 |
| **Extractibles (%)** | 6,4 | 5,7 |
| **Succion sous 2kPa (ml/g)** | 37 | 36 |
| **Succion sous 5kPa (ml/g)** | 23 | 28 |
| **Diamètre médian (um)** | 220 | 240 |
| **< 100 um (%)** | 8,4 | 8,0 |

## Revendications

1. Dans un procédé pour l'obtention de polymère d'acide polyacrylique sous forme de poudre superabsorbante pour les fluides aqueux, selon un processus comprenant une première étape de polymérisation d'une première charge d'acide acrylique monomère en phase aqueuse en suspension inverse dans un solvant organique en présence d'un émulsifiant d'HLB compris entre 8 et 12, puis une deuxième étape de polymérisation d'une deuxième charge préalablement absorbée au sein du gel polymère obtenu dans la première étape, un perfectionnement qui consiste :
- à introduire un agent de réticulation dans la première phase aqueuse d'acide acrylique,
- à utiliser une deuxième charge d'acide acrylique dans un rapport pondéral de 1 à 1,2 avec la première charge d'acide acrylique,
- à apporter au milieu réactionnel, après absorption de la deuxième charge de monomère et avant de provoquer sa polymérisation, un tensioactif d'HLB compris entre 2 et 5.

2. Procédé selon la revendication 1, caractérisé en ce que l'émulsifiant d'HLB compris entre 2 et 5 est le di/tristéarate de saccharose.

3. Procédé selon la revendication 1, caractérisé en ce que l'agent de réticulation est un dérivé bis acrylique ou un diglycidyléther.

4. Procédé selon la revendication 3, caractérisé en ce que l'agent de réticulation est le N,N-méthylènebisacrylamide.

5. Procédé selon la revendication 3, caractérisé en ce que l'agent de réticulation est l'éthylène glycol diglycidyléther.

6. Procédé selon l'une ou l'autre des revendications 1 à 5, caractérisé en ce dans la première étape au cours de laquelle on met une première charge d'acide acrylique monomère en phase aqueuse en suspension inverse dans un solvant organique, le rapport pondéral de la phase aqueuse à la phase organique va de 0,7 à 1,1.

7. Poudre superabsorbante telle qu'on peut l'obtenir par un procédé selon l'une ou l'autre des revendications précédentes et présentant les caractéristiques suivantes :
- un temps de prise en gel compris entre 15 et 40 secondes,
- une force de gel supérieure à 2.800 Pascals,
- une succion capillaire sous 2 kPa supérieure à 30 ml/g,
- une succion capillaire sous 5 kPa supérieure à 20 ml/g,
- une capacité d'absorption supérieure à 50g/g,
- un taux d'extractibles inférieur à 10 %,
- une teneur en fines inférieure à 10 %,
- une rétention supérieure à 28 g/g.

## Patentansprüche

1. Herstellung eines Acrylsäure-Polymeren in Form eines Pulvers, das superabsorbierende Eigenschaften für wäßrige Flüssigkeiten aufweist, nach einem Verfahren, das folgendes umfaßt: eine erste Polymerisationsstufe einer ersten Charge von monomerer Acrylsäure in wäßriger Phase in umgekehrter Suspension in einem organischen Lösungsmittel in Gegenwart eines Emulgiermittels mit einem HLB-Wert von 8 bis 12, anschließend eine zweite Polymerisationsstufe einer zweiten Charge, die vorwiegend im bei der ersten Stufe erhaltenen polymeren Gel absorbiert ist, wobei eine Verbesserung darin besteht, daß man:
- die erste wäßrige Phase von Acrylsäure mit einem Vernetzungsmittel versetzt,
- eine zweite Charge von Acrylsäure in einem Gewichtsverhältnis von 1 bis 1,2 zur ersten Acrylsäure-Charge verwendet und
- dem Reaktionsmedium nach der Absorption der zweiten Charge des Monomeren und vor der Herbeiführung von dessen Polymerisation ein oberflächenaktives Mittel mit einem HLB-Wert von 2 bis 5 zusetzt.

2. Verfahren nach Anspruch 1, dadurch gekennzeichnet, daß es sich beim Emulgiermittel mit einem HLB-Wert von 2 bis 5 um Saccharose-di/tristearat handelt.

3. Verfahren nach Anspruch 1, dadurch gekennzeichnet, daß es sich beim Vernetzungsmittel um ein Bisacrylderivat oder einen Diglycidylether handelt.

4. Verfahren nach Anspruch 3, dadurch gekennzeichnet, daß es sich beim Vernetzungsmittel um N,N-Methylenbisacrylamid handelt.

5. Verfahren nach Anspruch 3, dadurch gekennzeichnet, daß es sich beim Vernetzungsmittel um Ethylenglykoldiglycidylether handelt.

6. Verfahren nach einem der Ansprüche 1 bis 5, dadurch gekennzeichnet, daß in der ersten Stufe, in der man eine erste Charge von monomerer Acrylsäure in wäßriger Phase in eine inverse Suspension in einem organischen Lösungsmittel bringt, das Gewichtsverhältnis der wäßrigen Phase zur organischen Phase 0,7 bis 1,1 beträgt.

7. Superabsorbierendes Pulver, erhältlich nach einem Verfahren gemäß einem der vorstehenden Ansprüche, das folgende Eigenschaften aufweist:
- eine Gelbildungszeit von 15 bis 40 Sekunden,
- eine Gelstärke von mehr als 2800 Pascal,
- eine kapillare Saugwirkung bei 2 kPa von mehr als 30 ml/g,
- eine kapillare Saugwirkung bei 5 kPa von mehr als 20 ml/g,
- eine Absorptionskapazität von mehr als 50 g/g,
- einen Anteil an extrahierbaren Bestandteilen von weniger als 10%,
- einen Gehalt an Feinanteilen von weniger als 10% und
- eine Retention von mehr als 28 g/g.

## Claims

1. In a process for obtaining a polyacrylic acid polymer in superabsorbent powder form for aqueous fluids, according to a process comprising a first step of polymerization of a first acrylic acid monomer charge in aqueous phase in inverse suspension in an organic solvent in the presence of an emulsifier having an HLB of between 8 and 12, then a second step of polymerization of a second charge, absorbed beforehand within the polymer gel obtained in the first step, an improvement which consists:
- in introducing a crosslinking agent into the first aqueous phase of acrylic acid,
- in using a second acrylic acid charge in a ratio by weight of 1 to 1.2 to the first acrylic acid charge, and
- in adding to the reaction medium, after absorption of the second monomer charge and before initiating the polymerization thereof, a surfactant having an HLB of between 2 and 5.

2. Process according to Claim 1, characterized in that the emulsifier having an HLB of between 2 and 5 is sucrose di/tristearate.

3. Process according to Claim 1, characterized in that the crosslinking agent is a bisacrylic derivative or a diglycidyl ether.

4. Process according to Claim 3, characterized in that the crosslinking agent is N,N-methylenebisacrylamide.

5. Process according to Claim 3, characterized in that the crosslinking agent is ethylene glycol diglycidyl ether.

6. Process according to any of Claims 1 to 5, characterized in that, in the first step, during which a first acrylic acid monomer charge in aqueous phase is placed in inverse suspension in an organic solvent, the ratio by weight of the aqueous phase to the organic phase ranges from 0.7 to 1.1.

7. Superabsorbent powder obtainable by a process according to any of the preceding claims and exhibiting the following characteristics:
- a gelling time of between 15 and 40 seconds,
- a gel strength of more than 2,800 pascals,
- capillary suction under 2 kPa of more than 30 ml/g,
- capillary suction under 5 kPa of more than 20 ml/g,
- an absorption capacity of more than 50 g/g,
- a proportion of extractibles of less than 10 %,
- a fines content of less than 10 % and
- retention of more than 28 g/g.
